# EUROPÄISCHE PATENTANMELDUNG

(11) **EP 0 838 196 A2**
(43) Veröffentlichungstag der Anmeldung: **29.04.1998**
(21) Anmeldenummer: 97121037.2
(22) Anmeldetag: 30.11.1997
(51) Int. Cl.: A61B 17/00, A61B 17/60

(54) **Klemmverbindung für medizinische Geräte und Apparate**

(71) Anmelder: Spitzer, Daniel, D-07545 Gera (DE)
(72) Erfinder: Spitzer, Daniel, D-07545 Gera (DE)
(74) Vertreter: Amthor, Manfred W. J.

(57) **Zusammenfassung**

Die Erfindung betrifft die Kupplung zweier Seiten eines synergetisch zusammenwirkenden Fixationssystems bei medizinischen Geräten und Apparaten, dargestellt am Beispiel eines Fixateur externe mit stabartigen Führungselementen, wobei eine punktgenaue und intern spannungsfreie Klemmverbindung durch jeweils zwei planparallel angeordnete Flächen gebildet ist. Durch eine auf eine der Flächen fest aufgebrachte diamantene, plastische Körnungsschicht chaotischen Profils ist die komplementäre, unbeschichtete Kupplungsoberfläche bei jedem Kupplungsschluß durch eine statische Andrückkraft **"de novo" und "unikat"** formschlüssig geprägt, so daß eine Klemmverbindung mit multilatentem Kraft-Form-Schluß hergestellt ist.

## Beschreibung

Die Erfindung betrifft eine Klemmverbindung für medizinische Geräte und Apparate, dargestellt am Beispiel eines Fixateur extern mit einem Fixationsgerüst aus Führungsstäben. Fixateure dieser Art erfordern eine mechanische Verbindung zwischen den Führungsstäben und den Schanzschen Schrauben, die eine synergetische Kompatibilität dieser Systemseiten unter allen medizinischen und funktionalen Aspekten gewährleisten muß. Ein direkter Zugriff zu einer mehrachsig unbegrenzten Beweglichkeit bei intern spannungsfreier, punktgenauer und fester Kupplung von Führungsstäben des Fixationsgerüstes und der Schanzschen Schrauben ist das zentrale Kriterium socher Klemmverbindungen.

Die an eine solche Kupplung zu stellenden Anforderungen ergeben sich auch überwiegend aus der medizinischen Indikation des Fixateur externe.
So ist die externe Knochenbruchbehandlung mittels Schanzschen Schrauben bei I. - III.-gradig offenen Frakturen indiziert, da sie ein schnelles und vor allem minimal invasives und damit biologisches Verfahren ist, das die alles entscheidende verbliebene Vaskularisierung der geschädigten Extremität nur geringst möglich zusätzlich weiter iatrogen traumatisiert.
Da die Osteosynthesemethode des Fixateur externe außerdem ständig disponible interfrakturelle Interventionen wie Kompression, Distraktion, Neutralisation und Dynamisierung des repositionierten Bruches ermöglicht, ist sie zunächst nicht nur wegen der frakturfernen Lage der stabilisierenden Elemente und der minimierten Invasivität weit mehr als nur alternativ in Betracht gezogen.

Für den Fixateur externe sind daraus unverzichtbare konstruktive und funktionelle Anforderungen abgeleitet.

Zunächst verlangt die Phase der Frakturkonsolidierung eine absolut immobile Tetention des Repositionsergebnisses, da Bewegungen der Fragmente zu weitergehenden Weichteil- und Vaskularisierungsschäden führen.
Dies ist initial auch mit einer interne Spannungen enthaltenden und in ihrer Summe neutralen Fixateurkonstruktion möglich.
Im Verlaufe der Behandlung beginnen jedoch diese systeminternen Kräfte Effekte zu erzeugen, die sich primär an der Knochenkontaktzone der Schanzschen Schrauben bemerkbar machen. Spannungsindizierte Störungen der ossären Mikrozirkulation bewirken lokale Knochenresorptionen bis hin zur Sequestration mit konsekutiver Schanz'schraubenlockerung und obligater und sich perpetuierender Entzündungsreaktion als Schrittmacher von Weichteilinfektionen bis hin zur Osteomyelitis.
Weiterhin geht ein Verlust der Schraubenverankerung bei in der Regel noch nicht knöchern konsolidierter Fraktur auch mit einem Versagen der Osteosynthese einher.

Der Fixateur externe dieser Art muß ferner zwecks prozessualer Beschleunigung der interfrakturellen Konfiguration eine Druckstimulation durch eine Dynamisierung mittels teilweiser Aufgabe der streng axialen Fixationskomponenten zulassen.
Beide Qualitäten sind jedoch ohne zuverlässige Ausgangslage einer intern spannungs-neutralen Primärfixation nicht gesichert realisierbar.
Auch andere prozessuale Knochenstellungsänderungen oder - korrekturen unterliegen adäquaten Anforderungen an den Fixateur externe.

Die dem Stand der Technik bekannten Lösungen erfüllen diese Anforderungen jedoch nur unzureichend.
Die kontroverse Diskussion über einen Verfahrenswechsel auf innere Osteosynthese nach Weichteilkonsolidierung oder Benutzung des Fixateur externe als initiales und definitives Osteosyntheseverfahren belegt das.

Weitehin sind bei medizinischen Geräten und Apparaten ganz anderer Dimension, wie etwa Operationstischen, mechanische Klemmverbindungen von Bauteilen und Bauteiloberflächen in synergetisch inkompatibler Konstellation vorherrschender Stand der Technik. So funktionieren diverse Vorrichtungen der Operationstische zwecks präoperativer Lagemanipulation des Patienten mit formschlüssigen Profilkupplungen, die auf Grund der potentiell masseseitigen Anforderungen mit äußerst grober Teilung ausgerüstet sind. Der dadurch unvermeidbare grobe Lageversatz der beiden Kupplungsseiten bedingt unverträgliche Fehlpositionen des Patienten. Eine wünschenswert feinfühlige, genaue primäre Justierung, wie insbesondere auch notwendige akute Änderungen oder Korrekturen während der Operation, sind deshalb nicht möglich.
Die dagegen eingesetzten unzureichenden Provisorien bedeuten nicht nur breit angelegte praktische Nachteile des Handlings, sondern sind auch grundsätzlich inakzeptabel.

Für Klemmvervindungen medizinischer Instrumente, Geräte und Apparate der bezeichneten Art gilt deshalb die Forderung nach punktgenauer und intern kraftneutraler Fixation und Defixation bei minimierten Schließ- und Öffnungsmomenten, sowie gleichzeitig minimierten Schließ- und Öffnungswegen, wobei die generellen Forderungen nach geringer Eigenmasse und Baugröße, Einfachheit und dauerhafter Funktionssicherheit, Verschleißarmut oder praktischer Verschleißfreiheit, biologischer Dauerkompatibilität sowie klinisch bedenkenloser Handhabbarkeit, uneingeschränkt fortbestehen.

Das bei diesen medzinischen Geräten und Apparaten bisher völlig unbefriedigend behandelte und/oder vernachlässigte, überaus sensible und zentrale Problem, besteht somit im scheinbar unlösbaren geräteseitigen Zielkonflikt der physikalischen Beziehung zwischen Kraft und Weg; hier speziell bei einer kupplungsbildenden statischen Paarung zweier mechanischer Bauteile allein durch ihre Oberflächen.

Dabei bietet eine kraftschlußabbhängige Kupplungspaarung zweier völlig glatter und unprofilierter Flächen wohl eine ideal präzise und bewegungsfreie Fixierung bei geringstem Schließ- und Öffnungsweg, bedingt jedoch zur Erzielung ausreichender Belastbarkeit ein völlig unpraktikabel großes Schließ- und Öffnungsmoment, wodurch letztlich sogar die unmittelbare Kupplungspaarung selbst wieder zunehmend an Qualität einbüßt.

Der Stand der Technik zeigt in ähnlichem Zusammenhang durch EP 0 490 812 B1, Patentansprüche 6 und 7, sowie Zeichnung, Figur 1-13, eine geometrisch bedingte relative Vergrößerung der Paarungsoberfläche in Gestalt eines kugelförmigen Körpers, wobei eine der Oberflächen zusätzlich eine stoffeigene Profilierung durch sogenannte "Verriegelungsfasern", oder alternativ, einen "rutschfesten Überzug", trägt.
Beide Maßnahmen verbessern zwar die Haftung der beiden Paarungsoberflächen zueinander, was jedoch lediglich belegt, daß die bezeichnete Gelenkkupplung kraftschlüssig angelegt ist. Relativ hohe Schließ- und Öffnungskräfte sowie ein relativ großes Volumen der die Kupplungsflächen tragenden Bauteile bleiben deshalb prinzipiell unverzichtbar.

In naheliegendem Zusammenhang ist nach DE-OS 3722595 A1 eine "Vorrichtung zum externen Festlegen von Knochenfragmenten" bekannt geworden, die den unerwünschten "Klemmwiderstand" der beweglichen und verriegelbaren, als "Führungssäulen" des Fixateurs fungierende Teleskope, durch oberflächliche "Hartstoffbeschichtungen" außerordentlich großer Härte und niedrigen Reibkoeffizienten drastisch reduziert, wobei hierzu gemäß den Patentansprüchen 1 und 2 eine "diamantartige Kohlenstoffbeschichtung" vorgeschlagen ist.
Insoweit ist auch durch diese DE-OS auf die allgemeingültige dringende Grundforderung nach sensibler kinematischer "Gängigkeit" von Fixateuren deutlich hingewiesen, um "ruckartige Bewegungen", "die durchaus zu neuen Verletzungen im Bereich der Bruchstelle führen und den Heilungsprozeß verschlechtern können", auszuschließen.

Ein exponiertes Anliegen dieses Vorschlages gem. DE-OS 37 22 595 ist deshalb eine sichere und kontrollierte Nachstellung des Fixateurs unter Stauchbelastung, die nur unter stabil geringem internen Reibungseinfluß möglich ist. Übergreifend interessant ist der damit implizierte wesentliche Aspekt, daß die diamantene Hartstoffbeschichtung hier zur Verringerung interner Reibung gleitender Oberflächen medizinischer Geräte und Apparate eingesetzt ist, was ein breites, konkurrierendes Einsatzspektrum diamantener Beschichtungen ohne favorisierbare Priorität ausweist.
Auf das ältere Verfahren zur Herstellung der Hartstoffbeschichtung diamantartiger Kohlenstoffschicht gem. DE-PS 34 21 739 ist hingewiesen.

In der klinischen Praxis immer noch weit verbreitet sind ferner formschlüssige Paarungen der Oberflächen, die durch beidseitig eingearbeitete, kerbzahnähnliche, einander gegenüberliegend im Eingriff befindliche Profile, einen relativ festen und visuell kontrollierbaren Kupplungsverbund herstellen. Relativ geringe, wenngleich auch praktisch sehr differenzierte Schließ- und Öffnungskräfte, erfordern hier allerdings in Abhängigkeit von Profiltiefe und Flankenwinkel der Profilierung bedenklich große Schließ- und Öffnungswege, die zusätzlich und regelmäßig von einem Winkelversatz der Kupplungsoberflächen sowohl bei der Fixation, wie auch insbesondere der Defixation, bis etwa zur Größe einer ganzen Profilteilung begleitet sind.
/Hierzu PCT WO 92/13496, Fig. 3 und 4; ferner: DE-OS 38 02 833 A1, Fig. 2 sowie Spalte 3: "gezahnte Ringfläche 17"./, sowie insbesondere Zeitschrift OP-Journal Nr. 3/Jahrgang 11/Dezember 1995, S. 373, SYNTHES, Bilder zu "der kleine Fixateur"/

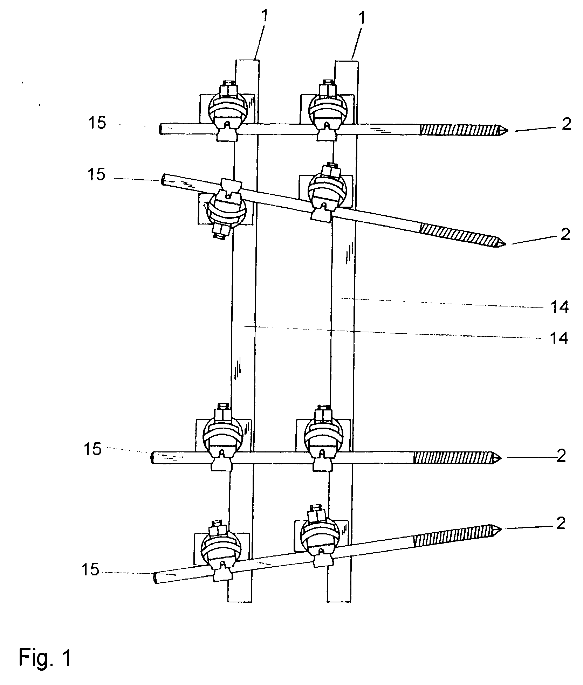

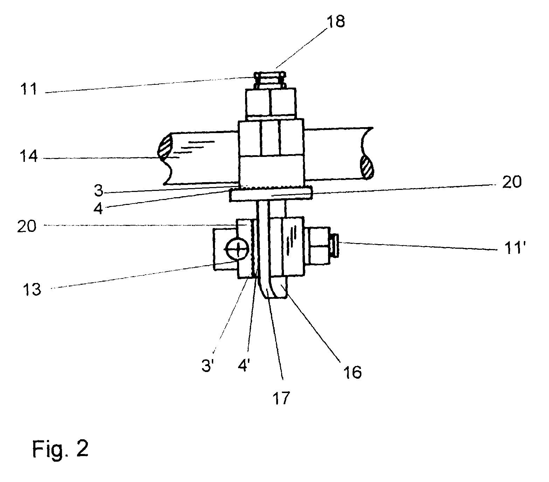

Gemäß DE-PS 37 27 400 C1 ist ferner im Zusammenhang mit der Fixierung sich tangierender zylindrischer Stäbe mit Außengewinde auf einen reinen Kraftschluß mit formschlüssiger Komponente verbal hingewiesen /Spalte 2, Zeile 13 - 28/, die lösungsgemäß nach Figur 1 und 2 jedoch lediglich eine geometrisch kritische Verspannung peripherer Gewindeabschnitte darstellt. Insoweit bleibt ihr verbaler Anspruch lösungsseitig eher ungedeckt.

Alle vorangehend zitierten, teilungsbedingten Formschlüsse funktionieren zwar ausreichend unter der Einwirkung legitimer externer Belastung, sind jedoch völlig unfähig, einen derartigen Formschluß unter Ausschluß illegitimer, kupplungsintern inizierter Belastungen, einzugehen.

Eine humanmedizinische Verträglichkeit dieser verzahnungsabhängigen, formschlüssigen Kupplungen ist deshalb für eine chirurgische Primärfixation im Zuge der Repositionierung, wie insbesondere eine sensible Defixation im Zuge postoperativer Dynamisierung, unter dem bereits beschriebenem Anspruch einer intern spannungsfreien und punktgenauen Kupplung grundsätzlich nicht erkennbar.

Auch insoweit erscheint es geboten, ein ganz offenbar verschüttetes Bedürfnis des Standes der Technik zu definieren und erfindungsmäß zu befriedigen.

Übereinstimmend sind alle vorbeschriebenen Lösungen des Standes der Technik bezüglich ihrer Verschlußkonfiguration kompromißorientierte Auswahlentscheidungen, die sich naturgemäß durch eine gerätespezifische Wichtung der verfügbaren Funktionskriterien und technischen Parameter auszeichnen und deshalb weder eine generell befriedigende Lösung, noch eine erkennbar konkrete Anregung für eine qualitativ weitergehende neue Lösung, liefern.

Im weiteren entfernten Zusammenhang mit der vorschlagsgemäßen Erfindung, ist auch auf längst vorbekannte manuelle Greifinstrumente, wie zweischenklige Pinzetten für medizinische, insbesondere chirurgische Zwecke, hinzuweisen, deren Innenseiten zur Verbesserung der Haft- und Greiffähigkeit sowie zur Verringerung der subjektiven Haltekraft mit einer diamantenen Körnungsschicht besetzt sind.
So zeigt bereits das DE-Gbm 1 794 844 eine "Pinzette für ärztlichen Gebrauch", bei der "die Innenflächen der vorderen Schenkelenden eine Diamantauflage oder eine dgl. Aufrauhung" aufweisen.
Die DE-OS 32 01 616 A1 beschreibt eine "Fremdkörperzange" mit zwei mit Diamantstaub plattierten oder überzogene Greifspitzen, um Fremdkörper, namentlich Glasstücke oder Glassplitter, bequem und mit leichter, minimaler Handbewegung, aus dem Körper eines Menschen oder Tieres zu entfernen. Ähnlich äußert sich auch das DE-Gbm 84 06 785.3., "Pinzette für chirurgische und kosmetische Zwecke". Schließlich beschreibt das DE-Gbm 85 12 734.5 eine "Präzisionszange", wobei zumindest eine der sich gegenüberliegenden Backeninnenseiten im Bereich ihrer Klemmzonen mit einer Körnungsschicht aus Diamant oder kubisch kristallinem Bornitrid ausgebildet ist, um die Klemmwirkung von mechanischen Aufrauhungen zu übertreffen und mit den Kornspitzen in die Oberfläche des Fixiergegenstandes einzudringen. Namentlich ist auf die Extraktion von Zähnen und Zahnwurzeln sowie die Gewebeentnahme, Fremdkörperentfernung und Knochenchirurgie verwiesen.
Abschließend resümiert das DE-Gbm, daß es der neuerungsgemäßen Vergütung des Zangenmauls im Bereich der Klemmzone auf eine sichere, verletzungsfreie Fixierung eines zu haltenden Teiles ankommt.

Allen zitierten Instrumenten ist somit gemeinsam, daß ihre beiden beschichteten Greifschenkel etwa im Bogenmaß eines bevorzugt gemeinsamen Schwenkradius beweglich sind und in finaler Operationsposition nahezu planparallel einen unabhängigen, regelmäßig nichtmetallischen Drittkörper, oberflächlich tangieren oder mit ihrer beidseitigen Körnungsschicht temporär durchdringen, um ihn nach manueller Manipulation zu entlassen.
Eine direkte und unmittelbare Flächenpaarung der beiden beschichteten metallischen Innenseiten, die das statuierte Prinzip instrumenter Greifkupplung verläßt, ist dabei unter Hinweis auf die konkrete Zweckbestimmung der Pinzetten ebenso absurd wie wertlos. Außerdem ist sie infolge beidseitiger Beschichtung der Schenkel, relativ elastischer Schenkelquerschnitte, sowie labilen, nicht spielfreien Schwenkmittelpunktes der Schenkel, zusätzlich technisch wirkungslos.
Solche weitergehenden Überlegungen zu einer radikalen Reduktion des beschriebenen Funktionsprinzips beschichteter Greifschenkel von Pinzetten im Sinne einer statischen Flächenpaarung ihrer beschichteten Oberflächen, etwa durch einen notwendig ersatzlosen Wegfall des unabhängigen Greifobjektes selbst, sind deshalb nicht nur nicht angeregt, sondern bereits im Ansatz unterdrückt oder abgewiesen.

Der Zugang zu solchen, bisher unbekannten Aspekten diamantbeschichteter planparalleler Oberflächen überhaupt, ist deshalb auch nur auf dem Wege einer nicht patenthindernden "Ex-post-facto-Analyse" unter wesentlichem Einbezug der antragsgemäßen Erfindung möglich. /EPA, T 564/89/

Die Aufgabe der Erfindung besteht deshalb darin, die dem Stand der Technik anhaftenden spezifischen Nachteile durch eine neu definierte, aus einem endlos wiederholbaren, punktgenauen Kraft-Form-Schluß komplementärer metallischer Oberflächen bestehenden Klemmverbindung, überraschend einfach und vollständig zu beseitigen, ohne dabei die dem Stand der Technik anhaftenden selektiven Vorzüge aufzugeben.

Erfindungsgemäß ist die Aufgabe in der Hauptsache durch eine Klemmverbindung gemäß Patentanspruch 1 dadurch gelöst, daß eine multilatente statische Prägekupplung mit teilungslosem Kraft-Form-Schluß aus zwei Kupplungsseiten 1, 2 mit direkt einander gegenüberliegend angeordneten Kupplungsscheiben 3, 4 metallischer Oberflächen 5, 6 besteht, derart, daß auf einer der Oberflächen 5 oder 6 eine dreidimensionale Körnungsschicht 7 aus Diamant oder kristallinem Bornitrid fest aufgebracht ist, so daß unter einer äußeren, auf beide Kupplungsscheiben 3, 4 wirkenden statischen Andrückkraft F die Kupplungsscheiben 3, 4 durch ihre Oberflächen 5, 6 teilungsslos kraftschlüssig, und, indem die unbeschichtete Oberfläche 5 oder 6 das jeweilige plastische Umkehrprofil 7' der dreidimensionalen Körnungsschicht 7 der beschichteten Oberfläche 5 oder 6 temporär annimmt, gleichzeitig teilungsslos formschlüssig kupplungsbildend sind.

Weitere Ausgestaltungen der die erfindungsgemäße Lösung tragenden Merkmale nach dem Hauptanspruch sind in den Unteransprüchen 2 bis 17 detailliert dargestellt.

Der Gegenstand der Erfindung und die erfindungsgemäße Lösung der Aufgabe sind nachfolgend am Beispiel eines Fixateur externe im einzelnen beschrieben.
Die dazugehörige Zechnung zeigt gemäß
- Figur 1:: eine Anzahl gleichartiger Klemmverbindungen der Kupplungsscheiben in der Umgebung von Kupplungsseiten eines Fixateur externe.
- Figur 2:: zwei in Reihe angeordnete Klemmverbindungen eines Fixateurs
- Figur 3:: eine Klemmverbindung mit fixierter Position einer Schanzschen Schraube
- Figur 4:: eine Klemmverbindung mit fixierter Position eines zylindrischen Führungsstabes und einer Schanzschen Schraube
- Figur 5:: die Kupplungsscheiben mit beschichteter und unbeschichteter Oberfläche und Druckfederelement

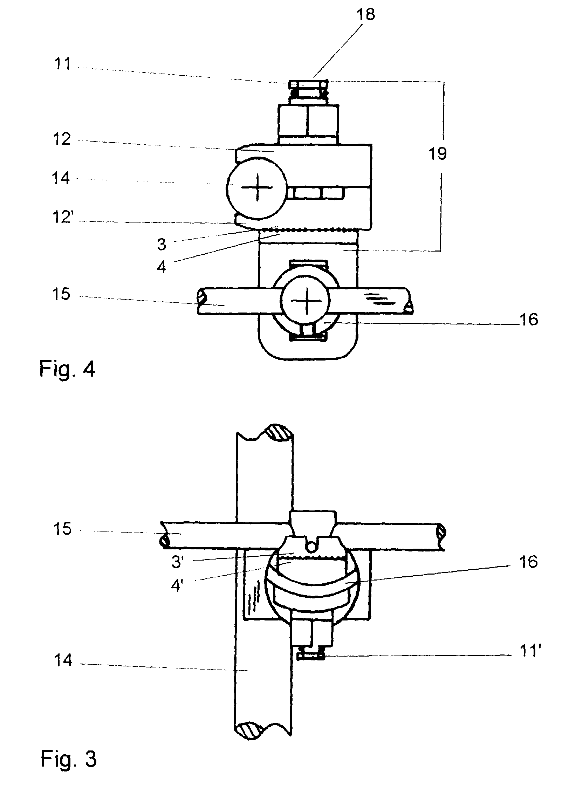

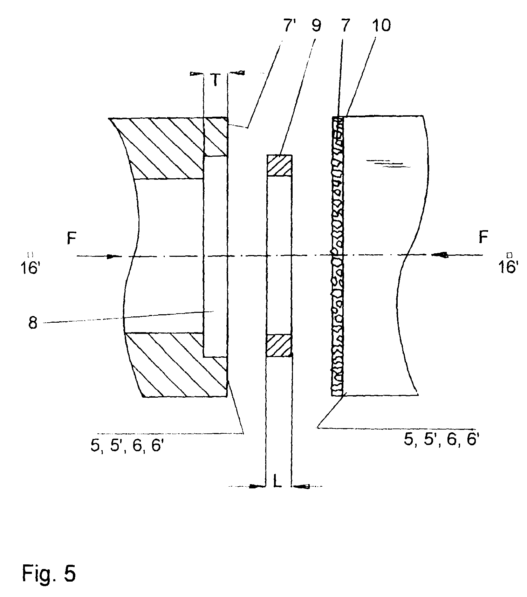

Die beiden Kupplungsseiten 1, 2 des Fixateur externe gem. Fig. 1, symbolisiert durch zwei in Reihe angeordenete, erfindungsbezogen adäquate Kupplungsscheibenpaare 3, 4, 3' und 4' gem. Fig 2, sind endseitig aus einer wählbaren Anzahl üblicher zylindrischer Führungsstäbe 14 einerseits und einer entsprechenden Anzahl üblicher Schanzschen Schrauben 15 andererseits gebildet.
Die offenen Klemmbacken 12 und 12' gem Fig. 3 und Fig. 4 sind, indem sie die Führungsstäbe 14 längsbeweglich teilweise umschließen, durch die Verschraubungen 19 mit den Führungsstäben 14 klemmbar verbunden.
Koaxial zu den Verschraubungen 19 der offenen Klemmbacken 12 und 12' trägt der zylindrische Stehbolzen 17 mehrachsig bewegliche mechanische Mittel 16 gem. Fig. 2, 3 und 4, die ihrerseits die Schanzschen Schrauben 15 in einer offenen Halbbohrung 13 spannbar aufnehmen. Jeweils eine der metallischen Oberflächen 5 oder 6 und 5' oder 6' der beiden Kupplungsscheibenpaare 3, 4 und 3', 4' sind mit einer diamantenen Körnungsschicht fest beschichtet, die unter der statischen, überwiegend nor-mal zu den Kupplungsebenen angreifenden Andrückkraft F gem. Fig. 5, der jeweils komplementären, unbeschichten Oberfläche 5 oder 6 und 5' oder 6', ihr plastisches Umkehrprofil 7' einprägt.
Die Körnungsschicht 7 ist dabei zweckmäßig auf eine Chrom-Nickel-Schicht aufgebracht.
Die jeweils unbeschichtete Oberfläche 5 oder 6 und 5' oder 6' ist schließlich mit einem zentrischen Innenbund 8 der axialen Länge T gem. Fig.5 ausgerüstet, in dem ein Druckfederelement 9 der entspannten Länge L zentrisch gelagert ist. Das Druckfederelement ist der über mechanische Mittel 16' abgestützten Andrückkraft F ständig abgemessen entgegengerichtet und unterstützt dadurch die unverzögerte Öffnung der Klemmverbindung nach Lösung der Verschlußmuttern 11 und 11'.

Durch diese Prägung ist eine punktgenaue, absolut spannungsfreie Klemmverbindung 20 hergestellt, wobei dieser multilatente Kraft-Form-Schluß praktisch verschleißfrei und endlos wiederholbar ist.
Das offene Gewindeende 18 des zylindrischen Stehbolzens 17 bleibt dabei für das Aufschrauben anderer oder zusätzlicher Muttern verfügbar.

Das Wesen der erfindinngsgemäßen Klemmverbindung für medizinische Geräte und Apparate besteht somit darin, daß die diamantene Körnungsschicht einer metallischen Oberfläche die jeweils autentische Umkehr ihres plastischen Profils unter einer etwa normal zur Flächenebene wirkenden statischen Kraft einer überwiegend planparallelen, unbeschichteten Oberfläche, formschlüssig und wiederholbar "de novo" einprägt.
Voraussetzungen einer komplikationsfreien externen Osteosynthese, insbesondere bei Fixateuren mit Führungsgerüsten zylindrischer Stäbe, sind damit erfindungsgemäß in einfacher und überaus praktikabler Form geschaffen.

### Verzeichnis der verwendeten Bezugszeichen

- 1: Kupplungsseite
- 2: Kupplungsseite
- 3: Kupplungsscheibe
- 4: Kupplungsscheibe
- 3': Kupplungsscheibe
- 4': Kupplungsscheibe
- 5: metallische Oberfläche der Kupplungsscheibe 3
- 6: metallische Oberfläche der Kupplungsscheibe 4
- 5': metallische Oberfläche der Kupplungsscheibe 3'
- 6': metallische Oberfläche der Kupplungsscheibe 4'
- 7: Körnungsschicht
- 7': plastisches Umkehrprofil der Körnungsschicht 7
- 8: zentrischer Innenbund
- 9: Druckfederelement
- 10: Chrom-Nickel-Schicht
- 11: Verschlußmutter
- 11': Verschlußmutter
- 12: offene Klemmbacke
- 12': offene Klemmbacke
- 13: offene Halbbohrung
- 14: zylindrischer Führungsstab
- 15: Schanzsche Schraube
- 16: mechanische Mittel
- 16': mechanische Mittel
- 17: zylindrischer Stehbolzen
- 18: offenes Gewindeende des zylindrischen Stehbolzens 17
- 19: Verschraubung
- F: statische Andrückkraft
- L: entspannte Länge des Druckfederelementes 9
- T: axiale Länge des zentrischen Innenbundes 8
- 20: Klemmverbindung der Oberfläche 5, 6, 5' 6' der Kupplungsscheiben 3, 4, 3' 4''

## Patentansprüche

1. Klemmverbindung für medizinische Geräte und Apparate, **gekennzeichnet** durch eine multilatente statische Prägekupplung mit teilungslosem Kraft-Form-Schluß, bestehend aus zwei Kupplungsseiten (1, 2) mit direkt einander gegenüberliegend angeordneten Kupplungsscheiben (3, 4) metallischer Oberflächen (5, 6), wobei auf eine der Oberfläche (5 oder 6) eine dreidimensionale Körnungsschicht (7) aus Diamant oder kristallinem Bornitrid fest aufgebracht ist, so daß unter einer äußeren, auf beide Kupplungsscheiben (3, 4) wirkenden statischen Andrückkraft (F), die Kupplungsscheiben (3, 4) durch ihre Oberflächen (5, 6) teilungslos kraftschlüssig, und , indem die unbeschichtete Oberfläche (5 oder 6) das jeweilige plastische Umkehrprofil (7') der dreidimensionalen Körnungsschicht (7) der beschichteten Oberfläche (5 oder 6) temporär annimmt, gleichzeitig teilungslos formschlüssig kupplungsbildend sind.

2. Klemmverbindung für medizinische Geräte und Apparate nach Patentanspruch 1, **dadurch gekennzeichnet**, daß die direkt gegenüberliegend angeordneten Oberflächen (5, 6) der Kupplungsscheiben (3, 4) konstant planparallel zueinander angeordnet sind.

3. Klemmverbindung für medizinische Geräte und Apparate nach den Patentansprüchen 1 und 2, **dadurch gekennzeichnet**, daß die Oberflächen (5, 6) der Kupplungsscheiben (3, 4) unprofiliert eben ausgebildet sind.

4. Klemmverbindung für medizinische Geräte und Apparate nach den Patentansprüchen 1 bis 3, **dadurch gekennzeichnet**, daß eine Oberfläche (5 oder 6) der Kupplungsscheibe (3 oder 4) kreisringförmig ausgebildet ist.

5. Klemmvervindung für medizinische Geräte und Apparate nach Patentanspruch 4, **dadurch gekennzeichnet**, daß die kreisringförmige Oberfläche (5 oder 6) der Kupplungsscheibe (3 oder 4) einen zentrischen Innenbund (8) aufweist, in dem ein normal zur Oberflächenebene wirkendes Druckfederelement (9) gelagert ist, dessen entspannte axiale Länge (L) größer ist als die axiale Länge (T) des zentrischen Innenbundes (8).

6. Klemmverbindung für medizinische Geräte und Apparate nach den Patentansprüchen 1-5, **dadurch gekennzeichnet**, daß die dreidimensionale Körnungsschicht (7) der Oberfläche (5 oder 6) größere Härte als die korrespondierende unbeschichtete Oberfläche (5 oder 6) der Kupplungsscheiben (3, 4) und eine plastische Oberfläche chaotischer Struktur aufweiset.

7. Klemmverbindung für medizinische Geräte und Apparate nach den Patentansprüchen 1-6, **dadurch gekennzeichnet**, daß mit den Kupplungsseiten (1 und/oder 2) korrespondierende mechanische Mittel (16) verbunden sind, deren Lage durch die kupplungsbildenden Oberflächen (3, 4) der Kupplungsscheiben (3, 4) exakt bestimmt ist.

8. Kemmverbindung für medizinische Geräte und Apparate nach Patentanspruch (7), **dadurch gekennzeichnet**, daß die mechanischen Mittel (16) aus offenen Klemmbacken (12, 12') und/oder offenen Halbbohrungen (13) gebildet sind, in denen parallel zur Kupplungsebene der Oberflächen (3, 4) bewegliche metallische Führungsstäbe (14) und/oder Schanzsche Schrauben (15) fixiert und befestigt sind.

9. Klemmverbindung für medizinische Geräte und Apparate nach den Patentansprüchen 1-8, **dadurch gekennzeichnet**, daß normal zur Kupplungsebene der Oberflächen (3, 4) verlaufende mechanische Mittel (16') mit einer auf die beiden Kupplungsscheiben (3, 4) gerichteten statischen Andrückkraft (F) angeordnet sind.

10. Klemmverbindung für medizinische Geräte und Apparate nach Patentanspruch 9, **dadurch gekennzeichnet**, daß die mechanischen Mittel (16') durch zylindrische Stehbolzen (17) mit offenem Gewindeende (18) und Verschraubung (19) gebildet sind.
